# EUROPEAN PATENT SPECIFICATION

(11) **EP 2 229 893 B1**
(45) Date of publication and mention of the grant of the patent: **10.07.2013**
(21) Application number: 10166547.9
(22) Date of filing: 05.12.2007
(51) Int. Cl.: A61B 17/06, A61B 17/00

(54) **Knotless wound closure device**
Knotenlose Wundverschlussvorrichtung
Dispositif de fermeture de plaies sans noeuds

(30) Priority: 05.12.2006 US 567129
(43) Date of publication of application: 22.09.2010
(62) Divisional of application: 07254703.7
(73) Proprietor: Covidien LP, Mansfield, MA 02048 (US)
(72) Inventor: Maiorino, Nicholas, Branford, CT 06405 (US); Primavera, Michael, Orange, CT 06477 (US); Buchter, Mark S, Ansonia, CT 06401 (US); Cohen, Matthew D, Berlin, CT 06037 (US)
(74) Representative: Soames, Candida Jane

(56) References cited:
- EP-A- 0 632 999
- EP-A- 1 669 093
- WO-A-01/52751
- US-A- 5 123 913
- US-A- 5 814 056

## Description

### TECHNICAL FIELD

The present disclosure generally relates to wound closure devices having an uninterrupted or continuous stitch.

### Background of Related Art

For many years, surgeons have sealed tissue wounds using various wound closure devices and methods. Suturing is a surgical technique involving the connection of tissue by stitching the tissue together with a strand of appropriate suturing material.

Typically, a suture is prepared by piercing a needle with the suture attached through tissue on both sides of a wound, by putting the ends of the suture to bring the sides of the wound together, and tying the suture into a knot. The knot preserves the tension on the suture to maintain the sides of the wound in approximation and allow the tissue to heal. An improperly tied knot can slip and untie at a tension far lower than the tension required to break the suture. When the suture is internal to the body, replacement of the failed suture can require another surgery.

A variety of devices have been developed for the transcutaneous placement, tying, and tightening of suture knots through a tissue tract. Despite the skill and due care involved in placing, tying, and tightening a suture knot using these devices, seepage of blood and fluids at the suture site and into the tissue tract can still occur.

Thus, there remains a need for fast and straightforward systems and methods to achieve wound cloture, which are substantially free of blood or fluid leakage about the wound closure site. There also remains a need for a wound closure device utilized as a continuous stitch which can have multi-use per patient instead of multiple sutures used for one wound closure. Furthermore, there still remains a need for a self anchoring wound closure device having a secure locking mechanism.

Document WO 01/052751 discloses a knotless wound closure device comprising an elongated flexible body and a plurality of through-holes formed along the length of the elongated flexible body, whereby pins are passed through the through-holes, and the pins are crimped or soldered for fixing so as to use the pins as substitutes for knots.

### SUMMARY

The present disclosure includes a knotless wound closure device as defined in claim 1.

The present disclosure also includes a method of closing a wound which includes using such wound closure devices by passing the proximal end of the device through body tissue at least once and subsequently passing the proximal end through at least one of the plurality of through-holes and forming a locked closed loop to secure body tissue held therein.

### BRIEF DESCRIPTION OF THE DRAWINGS

Various embodiments of the present disclosure will be described hereinbelow with reference to the figures wherein:
FIG. 1 is a perspective view of a knotless wound closure device attached to a needle in accordance with the present disclosure;
FIGS. 2A-D are top plan views of alternative embodiments of the plurality of through holes of FIG. 1 in accordance with the present disclosure;
FIGS. 3A-1 are cross sectional views of aternative embodiments of the elongated flexible body and surface features of the embodiment of FIG. 1 in accordance with the present disclosure; and
FIGS. 4-7 illustrate a series of steps employing the method of closing a wound in accordance with the present disclosure.

### DETAILED DESCRIPTION

Described herein are knotless wound closure devices utilized to form a continuous stitch and having an opposing anchoring point to function properly. The knotless wound closure device of the present disclosure eliminates the failure rate caused at or near the anchor point which would have caused support of the tissue approximation to be lost and seepage of blood and fluids to occur. The unique geometry and use of the knotless wound closure device also reduces or eliminates patient discomfort caused by the sharp protrusions which may be felt with a barbed suture device.

Accordingly, the knotless wound closure device is created with a specific geometry, namely with a plurality of surface features which interface with uniquely matching through-holes created through the length of the longitudinal axis of the elongated flexible body of the wound closure device which function as a unidirectional locking mechanism.

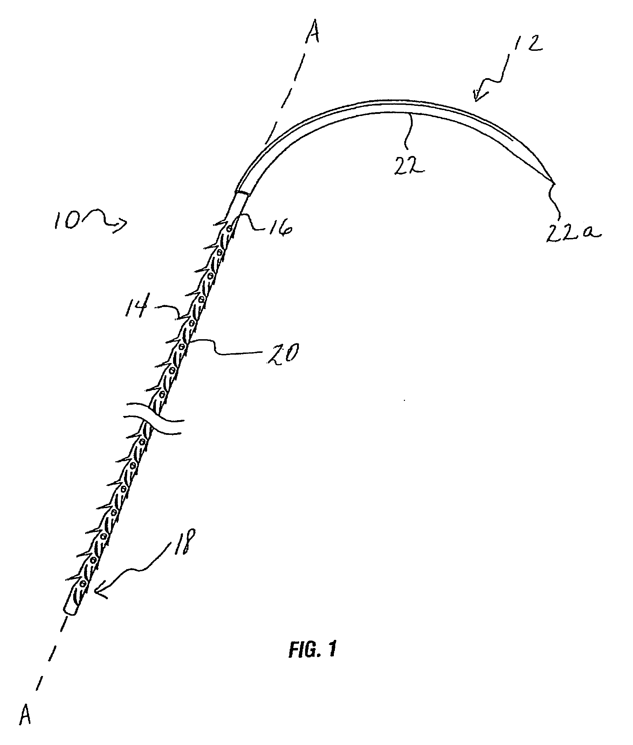

Referring now in detail to the drawings in which like reference numerals are applied to like elements in the various views, knotless wound closure device 10 is shown in FIG. 1 and generally includes an elongated flexible body 20 having a proximal end 12 with a needle 22 attached flexible body 20 and a distal end 18 and defining a longitudinal axis A-A. A plurality of surface features 14 extend generally away from the longitudinal axis A-A. Additionally, plurality of through-holes 16 are formed along the length of the elongated flexible body 20 wherein the needle 22 includes a sharpened tip 22a which is configured and dimensioned to pass through body tissue and thereafter be selectively passed through at least one of the plurality of through-holes 16 such that at least one of the surface features 14 also passes through the at least one through-hole 16 thereby forming a locked closed loop to secure body tissue held therein.

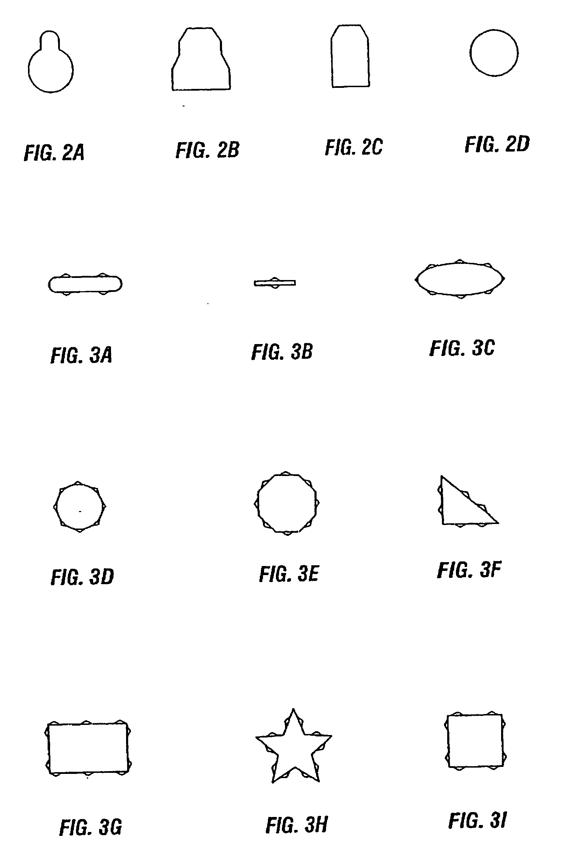

Referring to FIGS. 2A-D, in various alternative embodiments, the cross-sectional geometry of the plurality of through-holes 16 may consist of a key-shape (FIG. 2A), a compound wedge (FIG. 2B), a wedge (FIG. 2C) and a circle (FIG. 2D).

Referring now to FIGS. 3A-1, in various alternative embodiments, the cross-sectional geometry of the elongated flexible body 20 may consist of an oval shape (FIG. 3A), a flat tape shape (FIG. 3B), an elliptical shape (FIG. 3C), a round shape (FIG. 3D), an octagon shape (FIG. 3E), an oblique shape (FIG. 3F), a rectangle shape (FIG. 3G), a star shape (FIG. 3H) and a square (FIG. 3I).

In alternative embodiments, the surface features 14 may consist of barbs, hooks, latches, protrusions, leaves, teeth and/or combinations thereof.

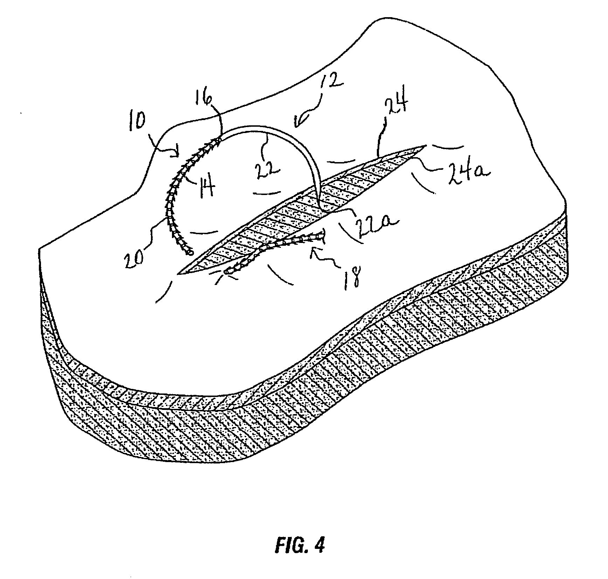

Referring to FIGS. 4-7, a series of steps or processes for an illustrative method of closing a wound using the knotless wound closure device described above is shown. In use, surface features 14 are interlaced through plurality of through-holes 16 penetrating through the longitudinal axis of elongated flexible body 20 and securing through a friction fit. Thus, as tension is applied to the device, a wedging action occurs thereby resulting in a more secure locking mechanism. The knotless wound closure device 10 and method of closing the wound is intended for general wound closure and can be utilize as either an "uninterrupted" or "continuous" stitch. The device will also support multi single unit use per patient.

In FIG.4, proximal end 12 of knotless wound closure device 10 is shown having penetrated both wound edges 24, 24a and approaching at least one of the plurality of through holes 15 of knotless wound closure device 10.

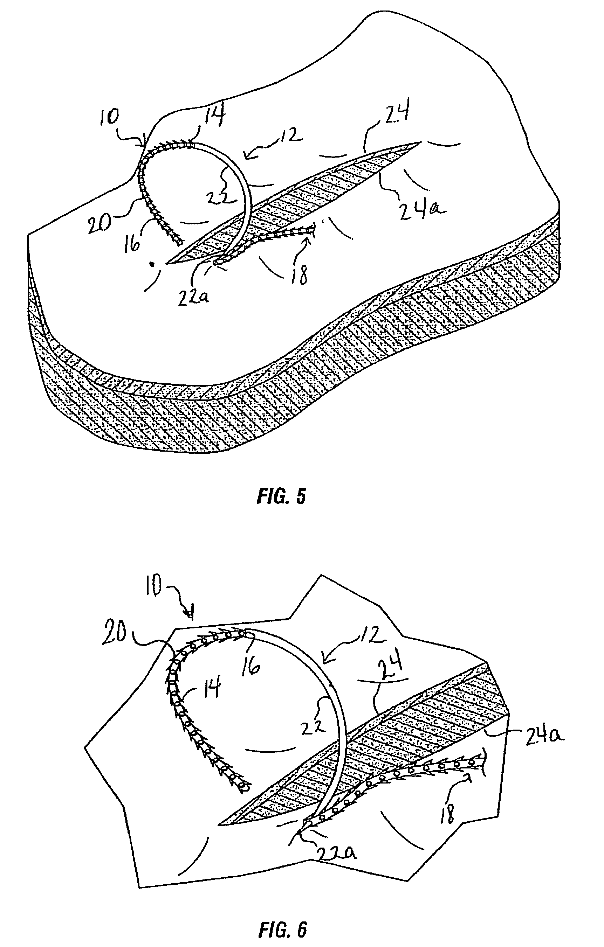

In FIG. 5, proximal end 12 is shown about to penetrate through one of the plurality of through holes 15.

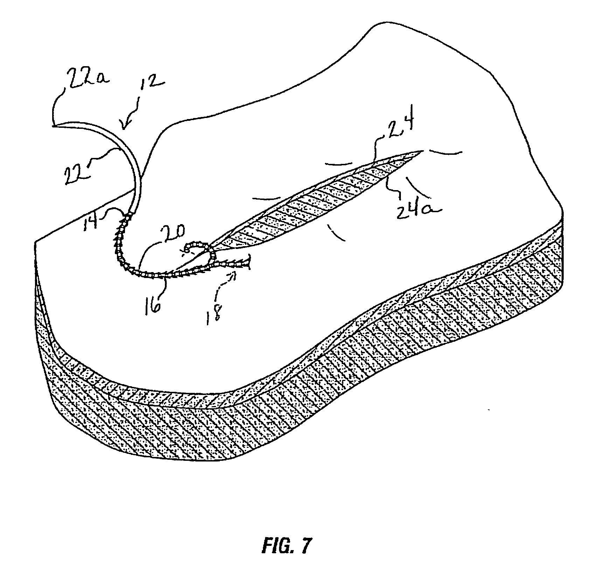

In FIG. 6, the proximal end 12 is shown penetrating through a through-hole 16. As proximal end 12 penetrates fully through the through-hole 16 (not shown) and the suture is pulled through through-hole 16, surface features 14 are compressed as they pass through through-hole 16 of knotless wound closure device 10 and expand upon exiting the other side of through-hole 16 thereby preventing reversal of the suture back through the through-hole 16. Additionally, the cross sectional dimension of flexible body 20 and the diameter of through hole 16 may be formed so as to create an interference or friction fit between the two thereby securing device 14 through a friction fit as shown in FIG. 7.

In various embodiments, the knotless wound closure device may be constructed from materials selected from the group consisting of surgical fibers, sutures, filaments, tapes, slit sheets, and ribbons.

In embodiments, a suture in accordance with the present disclosure may be of monofilament or multifilament construction. The suture may have both a proximal and distal end, with barbs projecting from the elongated body towards at least one end thereby forming an included angle of less than about 90 degrees between the barbs and the suture body. Additionally, a bioactive agent may be deposited within the barb angles, that is, the angle formed between the barb and suture surface. Placement of a bioactive agent in the angle formed between the barbs and suture surface places the bioactive agent at precisely defined locations within a tissue wound closure, which thereby provides a unique controlled and sustained release dosage form.

The wound closure device in accordance with the present disclosure may be formed of degradable materials, non-degradable materials, and combinations thereof. Suitable degradable materials which may be utilized to form the device include natural collagenous materials or synthetic resins including those derived from alkylene carbonates such as trimethylene carbonate, tetramethylene carbonate, and the like, caprolactone, dioxanone, glycolic acid, lactic acid, glycolide, lactide, homopolymers thereof, copolymers thereof, and combinations thereof. In some embodiments, glycolide and lactide based polyesters, especially copolymers of glycolide and lactide, may be utilized to form a suture of the present disclosure.

Suitable non-degradable materials which may be utilized to form the device of the present disclosure include polyolefins, such as polyethylene, polypropylene, copolymers of polyethylene and polypropylene, and blends of polyethylene and polypropylene; polyamides (also known as nylon); polyesters such as polyethylene terephthalate; polytetrafluoroethylene; polyether-esters such as polybutester; polytetramethylene ether glycol; 1,4-butanediol; polyurethanes; and combinations thereof. In other embodiments, non-degradable materials may include silk, cotton, linen, carbon fibers, and the like. In some useful embodiments, polypropylene can be utilized to form the suture. The polypropylene can be isotactic polypropylene or a mixture of isotactic and syndiotactic or atactic polypropylene.

Filaments used for forming wound closure devices of the present disclosure may be formed using any technique within the purview of those skilled in the art, such as, for example, extrusion, molding and/or solvent casting. In embodiments, the strands can be extruded through an extruder unit of a conventional type, such as those disclosed in U.S. Pat. Nos. 6,063,105; 6,203,564; and 6,235,869.

The device of the present disclosure may include a yam made of more than one filament, which may contain multiple filaments of the same or different materials. Where the device is made of multiple filaments, the suture can be made using any known technique such as, for example, braiding, weaving or knitting, each of which may be formed by any suitable method within the purview of those skilled in the art. The filaments may also be combined to produce a non-woven suture. The filaments themselves may be drawn, oriented, crinkled, twisted, commingled or air entangled to form yarns as part of the suture forming process.

Once the device is constructed, it can be sterilized by any means within the purview of those skilled in the art.

Wound closure devices in accordance with the present disclosure may be coated or impregnated with one or more medico-surgicatly useful substances, e.g., bioactive agents which accelerate or beneficially modify the healing process when the device is applied to a wound or surgical site. Suitable bioactive agents include, for example, biocidal agents, antibiotics, antimicrobial agents, medicants, growth factors, anti-clotting agents, analgesics, anesthetics, anti-inflammatory agents, wound repair agents and the like, and combinations thereof. Bioactive agents may be applied onto the wound closure device of the present disclosure utilizing any method within the purview of one skilled in the art including, for example, dipping, spraying, vapor deposition, brushing, compounding and the like.

Surface features may be formed on the elongated flexible body of the wound closure device utilizing any method within the purview of one skilled in the art. Such methods include, but are not limited to, cutting, molding, and the like. In some embodiments, surface features may be formed by making with acute angular cuts directly into the elongated flexible body, with cut portions pushed outwardly and separated from the body. The depth of the surface features thus formed generally away from the elongated flexible body may depend on the diameter of the material and the depth of the cut. In some embodiments, a suitable device for cutting a plurality of axially spaced surface features on the exterior of a filament may use a cutting bed, a cutting bed vise, a cutting template, and a blade assembly to perform the cutting. In operation, the cutting device has the ability to produce a plurality of axially spaced surface features such as barbs in the same or random configuration and at different angles in relation to each other. Other suitable methods of cutting the barbs include the use of a laser or manual methods. The device can be packaged in any number of desired pre-cut lengths and in pre-shaped curves.

In various embodiments, all of the surface features may be aligned to allow the elongated body of the knotless wound closure device to move through tissue in one direction and resist moving through tissue in the opposite direction. For example, referring to FIG. 1, the surface features 14 on elongated body 20 may be formed into a single directional wound closure device 10. In embodiments elongated body 20 may be attached to needle 22. The surface features 14 permit movement of device 10 through tissue in the direction of movement of a needle end 22 but are generally rigid in an opposite direction and prevent movement of device 10 in a direction opposite the direction of movement of a needle end 22.

In other embodiments, the surface features may be aligned on a first portion of a length of a body to allow movement of a first end of the device through tissue in one direction, while surface features on a second portion of the length of the body may be aligned to allow movement of the second end of the device in an opposite direction.

The surface features may be arranged in any suitable pattern, for example, in a helical pattern. The number, configuration, spacing and surface area of the surface features can vary depending upon the tissue in which the wound closure device is used, as well as the composition and geometry of the material utilized to form the wound closure device. Additionally, the proportions of the surface features may remain relatively constant while the overall length of the surface features and the spacing of the surface features may be determined by the tissue being connected. For example, if the wound closure device is to be used to connect the edges of a wound in skin or tendon, the surface features may be made relatively short and more rigid to facilitate entry into this rather firm tissue. Alternatively, if the wound closure device is intended for use in fatty tissue, which is relatively soft, the surface features may be made longer and spaced further apart to increase the ability of the wound closure device to grip the soft tissue.

The surface area of the surface features can also vary. For example, fuller-tipped surface features can be made of varying sizes designed for specific surgical applications. For joining fat and relatively soft tissues, larger surface features may be desired, whereas smaller surface features may be more suitable for collagen-dense tissues. In some embodiments, a combination of large and small surface features within the same structure may be beneficial, for example when a wound closure device is used in tissue repair with differing layer structures. Use of the combination of large and small surface features with the same wound closure device wherein barb sizes are customized for each tissue layer will ensure maximum anchoring properties. In certain embodiments, a single directional wound closure device as depicted in FIG. 1 may have both large and small surface features; in other embodiments a bi-directional wound closure device (not shown) may have both large and small surface features.

The wound closure device of the present disclosure may be utilized for all wound closure techniques and tissue connection procedures. Procedures can include endoscopic techniques, plastic and reconstructive surgeries, general wound closure, cardiovascular tissues, orthopedics, obstetrics, gynecology and urology. Typical tissue types include the various layers of muscle, ligaments, tendons, fascia, fat and/or skin.

There is also described a method of closing a wound comprising the steps of:
providing the knotless wound closure device as described herein and passing the proximal end of the knotless wound closure device through body tissue at least once and subsequently passing the proximal end through at least one of the plurality of through-holes thereby forming a locked closed loop to secure body tissue held therein.
The passing step may include passing the proximal end through body tissue a plurality of times before passing the proximal end through at least one of the plurality of through-holes thereby forming an uninterrupted stitch.

A knotless wound closure device includes an elongated flexible body with proximal and distal ends having a plurality of through-holes along its length and defining a longitudinal axis with a plurality of surface features extending away from the axis. The proximal end is configured and dimensioned to pass through body tissue and thereafter be selectively passed through at least one of the plurality of through-holes such that at least one of the surface features also passes through the through-hole thereby forming a locked closed loop to secure body tissue held therein.

While the above description contains many specifics, these specifics should not be construed as limitations on the scope of the disclosure, but merely as exemplifications of embodiments thereof. Those skilled in the art will envision many other possibilities within the scope of the disclosure as defined by the claims appended hereto.

## Claims

1. A knotless wound closure device comprising:
an elongated flexible body (20) having a proximal end (12) and a distal end (18), the elongated flexible body defining a longitudinal axis;
a plurality of surface features (14) formed along the length of the elongated flexible body and extending generally away from the longitudinal axis;
a plurality of through-holes (16) formed along the length of the elongated flexible body;
wherein the plurality of surface features (14) and the plurality of through-holes (16) are alternately positioned along the length of the flexible body; and
wherein the proximal end is configured and dimensioned to pass through body tissue and thereafter be selectively passed through at least one of the plurality of through-holes such that at least one of the surface features also passes through the at least one through-hole thereby forming a locked closed loop to secure body tissue held therein.

2. The knotless wound closure device of claim 1, wherein the device is constructed from materials selected from the group consisting of surgical fibers, sutures, filaments, tapes, slit sheets, and ribbons.

3. The knotless wound closure device of claim 1, wherein the cross-sectional geometry of the plurality of through-holes is selected from the group consisting of a key-shape, a wedge, a circle and a compound wedge.

4. The knotless wound closure device of claim 1, wherein the cross-sectional geometry of the elongated flexible body is selected from the group consisting of an oval, a rectangle, all ellipse, a circle, a square, a star, an oblique and an octagon.

5. The knotless wound closure device of claim 1, wherein the surface features are selected from the group consisting of barbs, hooks, latches, protrusions, leaves, teeth and/or combinations thereof.

6. The knotless wound closure device of claim 1, wherein the surface features are selected from the group consisting of barbs and hooks and/or combinations thereof.

7. The knotless wound closure device of claim 1, wherein the proximal end further comprises a needle (22) secured thereto.

8. The knotless wound closure device of claim 1, wherein the surface features includes a bioactive agent within an included angle of the surface feature and the elongated flexible body.

9. The knotless wound closure device of claim 8, wherein the bioactive agent is selected from the group consisting of biocidal agents, antibiotics, antimicrobial agents, medicaments, growth factors, anti-clotting agents, anesthetics, anti-inflammatory agents, wound repair agents and combinations thereof.

## Patentansprüche

1. Knotenlose Wundverschlussvorrichtung, die Folgendes umfasst:
einen länglichen flexiblen Körper (20), der ein proximales Ende (12) und ein distales Ende (18) hat, wobei der längliche flexible Körper eine Längsachse definiert,
eine Vielzahl von Oberflächeneigenschaften (14), die an dem Stück des länglichen flexiblen Körpers entlang gebildet sind und sich allgemein von der Längsachse weg erstrecken,
eine Vielzahl von Durchgangslöchern (16), die am Stück des länglichen flexiblen Körpers entlang gebildet sind,
wobei die Vielzahl der Oberflächeneigenschaften (14) und die Vielzahl der Durchgangslöcher (16) abwechselnd am Stück des flexiblen Körpers entlang positioniert sind, und
wobei das proximale Ende konfiguriert und dimensioniert ist, durch das Körpergewebe zu gehen und danach selektiv durch wenigstens eine der Vielzahl von Durchgangslöchern geführt zu werden, sodass wenigstens eine der Oberflächeneigenschaften auch durch das wenigstens eine Durchgangsloch geht und dabei eine verriegelte geschlossene Schleife zum Befestigen des Körpergewebes bildet, das darin gehalten wird.

2. Knotenlose Wundverschlussvorrichtung nach Anspruch 1, wobei die Vorrichtung aus Materialien gebildet ist, die von der Gruppe, die aus chirurgischen Fasern, Nahtfäden, Filamenten, Bändern, geschlitzten Bahnen und Streifen besteht, ausgewählt wird.

3. Knotenlose Wundverschlussvorrichtung nach Anspruch 1, wobei die Querschnittsgeometrie der Vielzahl von Durchgangslöchern von der Gruppe, die aus einer Schlüsselform, einem Keil, einem Kreis und einem Verbindungskeil besteht, ausgewählt wird.

4. Knotenlose Wundverschlussvorrichtung nach Anspruch 1, wobei die Querschnittsgeometrie des länglichen flexiblen Körpers von der Gruppe, die aus einem Oval, einem Rechteck, einer Ellipse, einem Kreis, einem Quadrat, einem Stern, einer Obliquität und einem Achteck besteht, ausgewählt wird.

5. Knotenlose Wundverschlussvorrichtung nach Anspruch 1, wobei die Oberflächeneigenschaften von der Gruppe, die aus Widerhaken, Haken, Klinken, Vorsprüngen, Blättern, Zacken und/oder Kombinationen davon besteht, ausgewählt wird.

6. Knotenlose Wundverschlussvorrichtung nach Anspruch 1, wobei die Oberflächeneigenschaften von der Gruppe, die aus Widerhaken und Haken und/oder Kombinationen davon besteht, ausgewählt wird.

7. Knotenlose Wundverschlussvorrichtung nach Anspruch 1, wobei das proximale Ende ferner eine Nadel (22) umfasst, die daran befestigt ist.

8. Knotenlose Wundverschlussvorrichtung nach Anspruch 1, wobei die Oberflächeneigenschaften einen bioaktiven Wirkstoff innerhalb eines eingeschlossenen Winkels der Oberflächeneigenschaft und dem länglichen flexiblen Körper beinhalten.

9. Knotenlose Wundverschlussvorrichtung nach Anspruch 8, wobei der bioaktive Wirkstoff von der Gruppe, die aus bioziden Wirkstoffen, Antibiotika, antimikrobiellen Wirkstoffen, Medikamenten, Wachstumsfaktoren, gerinnungshemmenden Wirkstoffen, Anästhetika, entzündungshemmenden Wirkstoffen, Wundheilungswirkstoffen und Kombinationen davon besteht, ausgewählt wird.

## Revendications

1. Dispositif de fermeture de plaies sans noeuds,
comprenant :
un corps flexible allongé (20) ayant une extrémité proximale (12) et une extrémité distale (18), le corps flexible allongé définissant un axe longitudinal ;
une pluralité de saillies de surface (14) formées sur la longueur du corps flexible allongé et s'écartant de manière générale de l'axe longitudinal ;
une pluralité de trous traversants (16) formés sur la longueur du corps flexible allongé ;
dans lequel la pluralité de saillies de surface (14) et la pluralité de trous traversants (16) sont positionnés en alternance sur la longueur du corps flexible ; et
dans lequel l'extrémité proximale est configurée et dimensionnée pour passer à travers un tissu corporel et, ensuite, passer sélectivement à travers au moins l'un de la pluralité de trous traversants de sorte qu'au moins l'une des saillies de surface passe également à travers le au moins un trou traversant, formant ainsi une boucle fermée bloquée afin de fixer le tissu corporel qui y est retenu.

2. Dispositif de fermeture de plaies sans noeuds selon la revendication 1, dans lequel le dispositif est conçu à partir de matériaux choisis dans le groupe constitué des fibres chirurgicales, des sutures, des filaments, des bandes adhésives, des feuilles incisées et des rubans.

3. Dispositif de fermeture de plaies sans noeuds selon la revendication 1, dans lequel la géométrie en coupe transversale de la pluralité de trous traversants est choisie dans le groupe constitué d'une forme de clé, d'un coin, d'un cercle et d'un coin composite.

4. Dispositif de fermeture de plaies sans noeuds selon la revendication 1, dans lequel la géométrie en coupe transversale du corps flexible allongé est choisie dans le groupe constitué d'un ovale, d'un rectangle, d'une ellipse, d'un cercle, d'un carré, d'une étoile, d'une oblique et d'un octogone.

5. Dispositif de fermeture de plaies sans noeuds selon la revendication 1, dans lequel les saillies de surface sont choisies dans le groupe constitué de barbes, de crochets, de verrous, de saillies, de lamelles, de dents et/ou de combinaisons de ceux-ci.

6. Dispositif de fermeture de plaies sans noeuds selon la revendication 1, dans lequel les saillies de surface sont choisies dans le groupe constitué de barbes et de crochets et/ou de combinaisons de ceux-ci.

7. Dispositif de fermeture de plaies sans noeuds selon la revendication 1, dans lequel l'extrémité proximale comprend en outre une aiguille (22) qui lui est fixée.

8. Dispositif de fermeture de plaies sans noeuds selon la revendication 1, dans lequel les saillies de surface comprennent un agent bioactif dans un angle aigu formé entre la saillie de surface et le corps flexible allongé.

9. Dispositif de fermeture de plaies sans noeuds selon la revendication 8, dans lequel l'agent bioactif est choisi dans le groupe constitué des agents bio-acides, des antibiotiques, des agents antimicrobiens, des médicaments, des facteurs de croissance, des agents anticoagulants, des anesthésiques, des agents anti-inflammatoires, des agents de réparation de plaies et de leurs combinaisons.
